# EUROPEAN PATENT SPECIFICATION

(11) **EP 0 479 899 B1**
(45) Date of publication and mention of the grant of the patent: **06.03.1996**
(21) Application number: 90911134.6
(22) Date of filing: 29.06.1990
(51) Int. Cl.: C07K 14/435

(54) **VASODILATORY AND IMMUNE SUPPRESSANT PEPTIDES**
VASODILATIERENDE UND IMMUNSUPPRIMIERENDE PEPTIDE
PEPTIDES VASODILATATEURS IMMUNOSUPPRESSEURS

(30) Priority: 29.06.1989 US 374080
(43) Date of publication of application: 15.04.1992
(73) Proprietor: THE PRESIDENT AND FELLOWS OF HARVARD COLLEGE, Cambridge, MA 02138 (US)
(72) Inventor: LERNER, Ethan, A., Brookline, MA 02146 (US); REMOLD, Heinz, G., Brookline, MA 02146 (US); RIBEIRO, Jose, M., C., Winchester, MA 01890 (US); TITUS, Richard, G., Needham, MA 02192 (US)
(74) Representative: Holdcroft, James Gerald, Dr.
(86) International application number: US9003746
(87) International publication number: WO9100293

(56) References cited:
- Science, vol. 243, January 1989, J M C Riberio et al.:"A novel vasodilatory peptide from the salivary glands of the sand fly Lutzomyia Longipalpis", see page 212-214
- Science, vol. 239, March 1988, R G Titus et al.:"Salivary Gland Lysates from the sand fly Lutzomyia Longipalpis enhance Leishmania infectivity", see page 1306-1308

## Description

The United States government may have rights to this invention pursuant to NIH grant numbers AI24511, AI18694, and AI22794.

### Background of the Invention

This invention relates to proteins capable of inducing vasodilation and temporary immune suppression in mammals, to compositions containing such proteins, to methods for producing the proteins using peptide synthesis and recombinant DNA techniques, and to synthetic forms of such proteins. The invention also relates to a method of desensitizing a mammal to the effects of an immunogen by administering certain peptides which temporarily inactivate the immune system.

Vasodilators are drugs useful in the treatment of various conditions characterized by constricted blood vessels. Such conditions include Raynaud's syndrome, certain post surgical complications of brain surgery involving sub arachnoid hemorrhage, heart failure, angina pectoris, and hypertension. Recently, the neuropeptide calcitonin gene-related peptide (CGRP) has been characterized as the most powerful and persistent vasodilator known. Calcitonin, another neuropeptide known primarily for its ability to prevent bone loss during periods of calcium stress, is derived from the same gene as CGRP. Despite weak structural homologies, there is enough similarity in the conformations of calcitonin and CGRP that they interact at each other's receptors. Thus, CGRP has a weak calcitonin-like effect on bone (Zaidi et al., quart. J. Exp. Physiol. 72:371 (1987)).

Ribeiro et al. (1989), Science, Vol 243, pp. 212-214 describe a vasodilatory peptide from the Sand Fly Lutzomyia longipalpis, and present some preliminary data which indicate that the peptide has vasodilatory activity.

The macrophage, a large phagocytic cell of the reticuloendothelial system, plays a central role in the induction and expression of cellular immunity. Antigen processing and subsequent presentation of antigen by macrophages in the presence of class II histocompatibility antigens can trigger helper T-lymphocyte response (Buss et al., Immunol. Rev. 98: 115 (1987). In addition, macrophages can control T-cell responses via production of cytokines such as IL-1 (Unanue et al., Ann. L'institute Pasteur 138: 489 (1987)).

Activation of macrophages enhances the microbicidal and tumoricidal activity of the cells, an event which is paralleled by significant changes in the levels of various intracellular, secreted and cell surface proteins (Adams et al., Ann. Rev. immunol. 2: 283 (1984)). For example, levels of secreted IL-1 and expressed class II histocompatability antigen rise, (Adams et al., ibid.) while the level of 5'nucleotidase has been shown to fall (Johnson et al., J. Immunol. 131: 1038 (1983)). In addition, the production of H₂O₂ by activated macrophages is increased over controls (Adams et al., ibid.). Macrophages can be activated by a number of lymphokines such as IFN-γ (Merry et al., J. Immunol. 134: 1619 (1985)), and by bacterial cell wall products such as lipopolysaccharide (Pabst et al., J. Exp. Med. 151: 101 (1980)). Recently, it has been suggested that as activated macrophages sterilize the site of inflammation they are deactivated so as to avoid possible damage to host tissue via continued release of cytotoxic products. (Tsunawaki et al., Nature 334: 260 (1988)).

A first object of this invention is to provide proteins derived from the salivary lysate of the sand fly Lutzomyia longipalpis capable of vasodilation and of temporary immune suppression in mammals. Other objects are to characterize the protein, to provide natural and recombinant forms of the protein, and to provide genes encoding the protein and methods for production of the protein using recombinant DNA and peptide synthesis techniques. A second object is to provide methods for desensitizing a mammal to the effects of an immunogen by administering the protein derived from Lutzomvia, CGRP, calcitonin, active analogs, or synthetic forms thereof.

### Summary of the Invention

It has now been discovered that a protein derivable from the salivary gland lysate of the sand fly Lutzomyia longipalpis is capable of inducing vasodilation and/or temporary immune suppression in mammals. In one embodiment, the protein is substantially pure protein, or an active fragment thereof, derivable from the salivary gland lysate of the sand fly Lutzomyia longipalpis which protein induces vasodilation or temporary immunosuppression in a mammal and has a molecular weight of about 6839 daltons as determined by mass spectrometry. The protein may be further characterized as eluting prior to CGRP in an acetonitrile-H₂0-trifluoracetic acid-reverse phase-high performance liquid chromatography column, or having vasodilation activity as measured by erythema induction in animal skin at least about 80-100 times that of CGRP, or constituting about 1% of the total protein in the salivary glands of said fly. Like CGRP, the vasodilation activity may persist for relatively long periods, e.g. several days.

Temporary immune suppression induced by this protein takes the form of inhibition of macrophage function as indicated by prevention of increase of H₂O₂ production by γ IFN and by suppression of the macrophage's ability to present antigen to T-cells. It is believed that one active protein is responsible for both the vasodilation and immune supression activities.

The protein ("Lutzomyia Protein" or "LP") can be derived from lysate of the salivary glands of the sand fly by chromatographic purification as disclosed herein. The nucleotide sequence of a gene encoding LP has been determined and the amino acid sequence deduced. A second DNA sequence encoding LP has also been identified which varies somewhat from the first sequence determined both in terms of nucleotide sequence and the deduced amino acid sequence. It appears, therefore, that there are two or more variants of LP. The protein and various active analogs and fragments thereof can be produced by expression of recombinant DNA in a host call or by peptide synthesis techniques. Compositions rich in LP or its active analogs and fragments may be used pharmaceutically as an immune system supressing drug or as a potent vasodilator. The active analogs and fragments of LP are typically proteins or peptides comprising an amino acid sequence sufficiently duplicative of the sequence of the active portion of an LP protein such that the proteins or peptides are capable of inducing vasodilation or temporary immune suppression in a mammal.

Thus, in another aspect, the invention comprises the protein of the invention for use in therapy, e.g. in a method of increasing blood flow in the circulatory system of a mammal by administering to the mammal an effective amount of LP, or active analog or fragment thereof, to cause vasodilation in the mammal. Parenteral administration can result in systemic vasodilation activity. Topical application, e.g., to a vascular bed during surgery, can serve to concentrate the vasodilatory effect in the locus of application.

In another aspect, it has been discovered that, in addition to LP, the structurally related CGRP and calcitonin peptides also can be used to supress the immune system temporarily. The invention thus further provides the use of these proteins for the manufacture of a medicament for desensitizing a mammal to the effects of an immunogen by temporarily suppressing the immune system of the mammal during exposure to the immunogen, the immunogen being for example an immunogen derived from a source xenotypic to said mammal, e.g. in man, streptokinase or a mouse monoclonal antibody. Thus, for example, these temporary immune suppressing substances may be administered in conjunction with a protein xenotypic to the mammal (such as streptokinase or a murine monoclonal in man so as to inhibit or prevent the development of antibodies or cellular immunity to the drug. The immune suppressing substances may also be used to treat graft rejection and autoimmune disease.

### Brief Description of the Drawing

The foregoing and other objects and features of the invention, as well as the invention itself, may be more fully understood from the following description, read together with the accompanying drawings, in which:
FIGURE 1 shows a reverse-phase HPLC chromatogram of salivary gland extract;
FIGURE 2 shows the results of capillary electrophoresis of reverse-phase HPLC purified LP;
FIGURE 3 is a graph showing the potency and persistence of erythema induced by LP and CGRP;
FIGURE 4 is a graph representing the level of relaxation of a constricted rabbit aortic ring by Lutzomyia longipalpis salivary gland lysate measuring tension vs. time; A indicates addition of the vasoconstrictor adrenalin; S indicates addition of salivary gland lysates; W represents washing of the preparation; the second A represents a second addition of adrenalin;
FIGURE 5 is a bar graph showing the effect of CGRP on H₂O₂ production of human macrophages pretreated with Interferon-γ; the bars represent mean H₂O₂ production for triplicate cultures ± standard deviation (SD); and
FIGURE 6 is a graph comparing the effect of two neuropeptides, calcitonin (Δ) and CGRP (Δ) on macrophage function (vertical bars equal standard deviation).

### Description

An active protein (LP) in the saliva lysate of the sand fly Lutzomyia longipalpis has been discovered to be a potent vasodilator. In addition, it has been discovered that calcitonin, CGRP and LP all exhibit an ability to suppress temporarily the immune system of mammals.

LP can be obtained by conventional purification chromatography from surgically excised salivary glands of L. longipalpis as disclosed below. One pair of salivary glands contains 10-15ng LP.

The nucleotide sequence which codes for LP and the amino acid sequence of LP are given in the sequence listing at page 26. Knowledge of the LP sequence enables skilled engineers to produce large quantities of the protein for therapeutic use. The artisan can synthesize LP or active analogs thereof using conventional chemical solid or solution phase peptide synthesis techniques. In addition, knowledge of the sequence permits expression of DNA sequence coding for LP or active analogs or fragments thereof in various types of host cells, including both procaryotes and eucaryotes, to produce large quantities of the protein, or active analogs or fragments thereof, and other constructs capable of inducing vasodilation or temporary immune suppression in a mammal.

The compounds of the present invention can be formulated into pharmaceutical preparations for therapeutic use. In particular, LP can find use as a therapeutic vasodilating agent and consequently as a regulator of blood pressure. Also, CGRP and calcitonin (both available commercially) as well as LP may be used to induce temporary immune suppression.

These compounds can be administered to mammalian hosts for veterinary use such as with domestic animals, and for clinical use in humans in a manner similar to other therapeutic agents. In general, the dosage will range from about 2pg to 0.25µg per kg of host body weight. Dosages within these ranges can be used in an amount per administration which may vary depending on the severity of the condition treated until benefits have been obtained. The protein can be injected intravascularly to provide a systemic vasodilation effect to treat, for example, Raynaud's syndrome. It also may be applied topically or by infusion to induce locally a vasodilatory action, for example, during brain surgery to alleviate blood vessel constriction and subsequent brain damage. These compounds may be formulated for oral, buccal, parenteral, or rectal administration or in a form suitable for nasal administration or administration by inhalation or insufflation.

These compounds can be administered neat, as mixtures with other pharmacologically active or inactive materials, or with physiologically suitable carriers such as, for example, water, normal saline, or buffers compatible with physiological conditions. Injection can be subcutaneous, intravenous, or intramuscular. These compounds may be administered as pharmacologically acceptable salts such as acid addition salts. The protein may be stored in lyophilized form and reconstituted just prior to use.

The subject matter claimed herein will be further understood from the following.

### Isolation of LP

Sand flies were reared from a laboratory strain of Lutzomyia longipalpis originally provided by the Walter Reed Army Institute of Research following the procedure described by Modi et al., J. Med. Entomol. 20: 568-570 (1983). Wild specimens can be captured in various tropical regions in South America. Larvae were fed a mixture of fermented rabbit feed (Purina), rabbit faeces, and liver powder. Adults were kept at 100% relative humidity and were given free access to a saturated sucrose solution. Salivary glands are dissected from 5-7 day old female flies and stored in phosphate-buffered saline containing 1 mg/ml bovine serum albumin (PBS-BSA). The glands are excised using a #5 forceps and surgical needles to remove the prominent glands posterior to the head. Pairs of glands are transferred to 20 µl of Tris-HCl buffer, 5mM, pH 7.4, and may be frozen at -70°C until needed.

### Characterization of LP

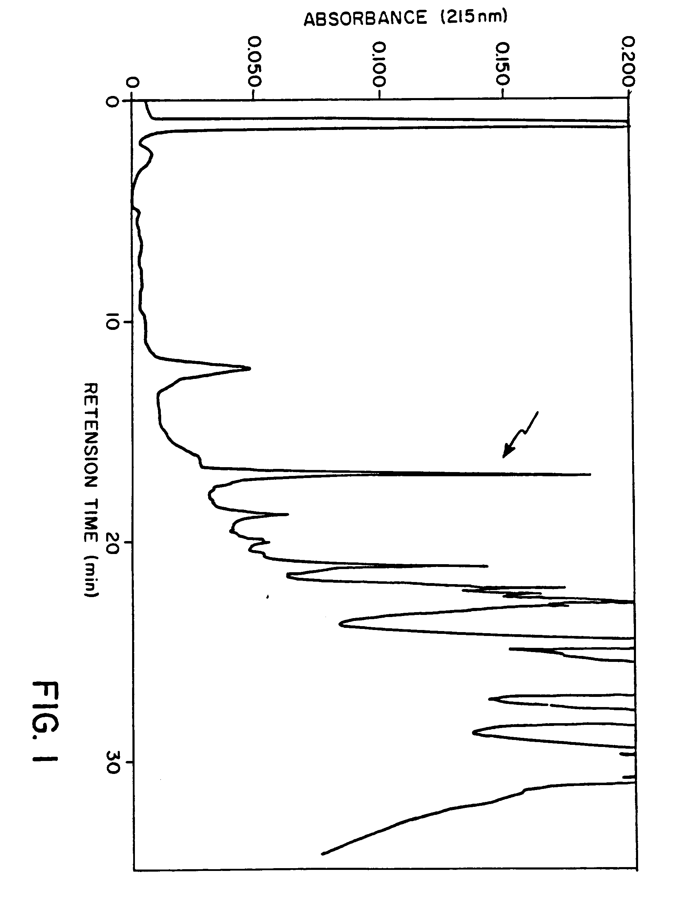

Reversed-phase high-performance liquid chromatography (HPLC) on the extract of 105 pairs of salivary glands is depicted in Figure 1. 105 pairs of salivary glands were dissected in PBS. Lysates were made by freeze-thawing. The extract was spun in a microcentrifuge for 30 seconds before being applied to the column. A C-18 micropellicular HPLC column (Glycotech) was equilibrated with 20% acetonitrile/0.1% trifluoroacetic acid at 50°C at a flow rate of 0.6ml/minute. Three minutes after injection, a linear gradient was run for 20 minutes reaching 44% acetonitrile. Fractions were collected every 30 seconds and assayed for vasodilator activity. The vasodilator assay was performed by diluting material from the gradient 10 fold and injecting 50 µl intradermally into the shaved back of a rabbit. The development of erythema at the injection site corresponded with the peak indicated. One µg of human CGRP, used as a control peptide, eluted as a peak of equal area, five minutes later (not shown). This observation implies that approximately ten nanograms of LP are present per pair of salivary glands.

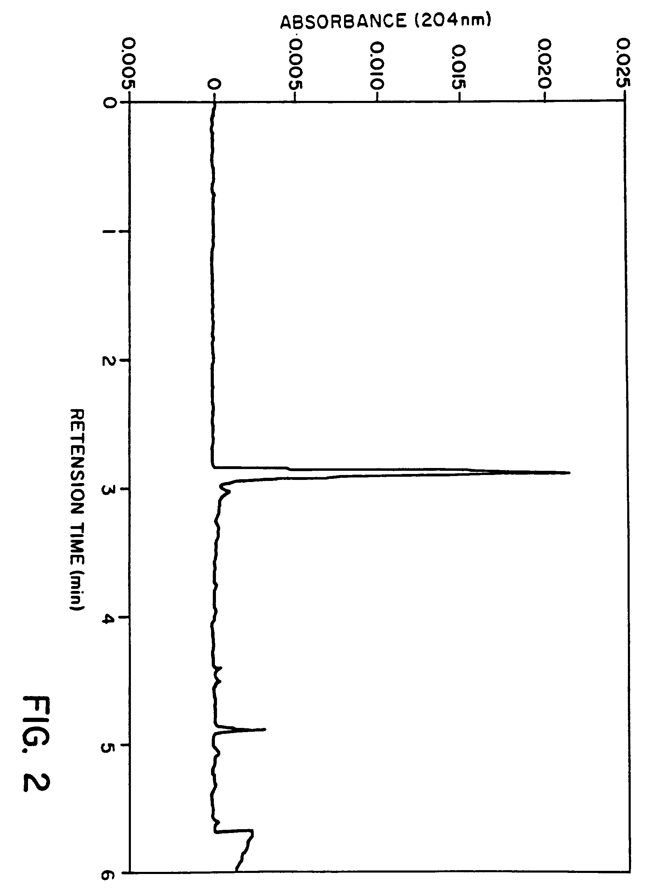

Because multiple components could be hidden under a single peak from HPLC, an independent measure of LP purity was needed. Capillary electrophoresis, which can separate molecules on the basis of charge, was chosen. The limitation here was the need to have a volume of a few microliters where the concentration of LP would approximate the lmg/ml used in capillary electrophoresis. 250 pairs of salivary glands were dissected and 2.5 µg of LP isolated from multiple runs on RP-HPLC. The fractions containing LP were pooled, yielding a volume of approximately two ml. LP was concentrated in a Speed-Vac to a volume of 10 µl in preparation for capillary electrophoresis. Sample was injected for three seconds at 1/2 psi and electrophoresed at 25 kv on a Beckman P/ACE 2000 instrument. A 100 mM borate buffer of pH 8.3 was chosen because isoelectic focusing of the HPLC-purified EIP revealed activity at a pI of 7.8. The sloping peak near six minutes represents trifluoroacetic acid remaining from the HPLC run. A large peak was present after nearly three minutes of electrophoresis (Figure 2).

0.5 microgram of RP-HPLC purified LP was analyzed via fast-atom-bombardment (FAB) mass spectrometry in order to determine its mass. A single component of 6839 mass units was detected. The mass of LP is thus different from that of 3900 for CGRP.

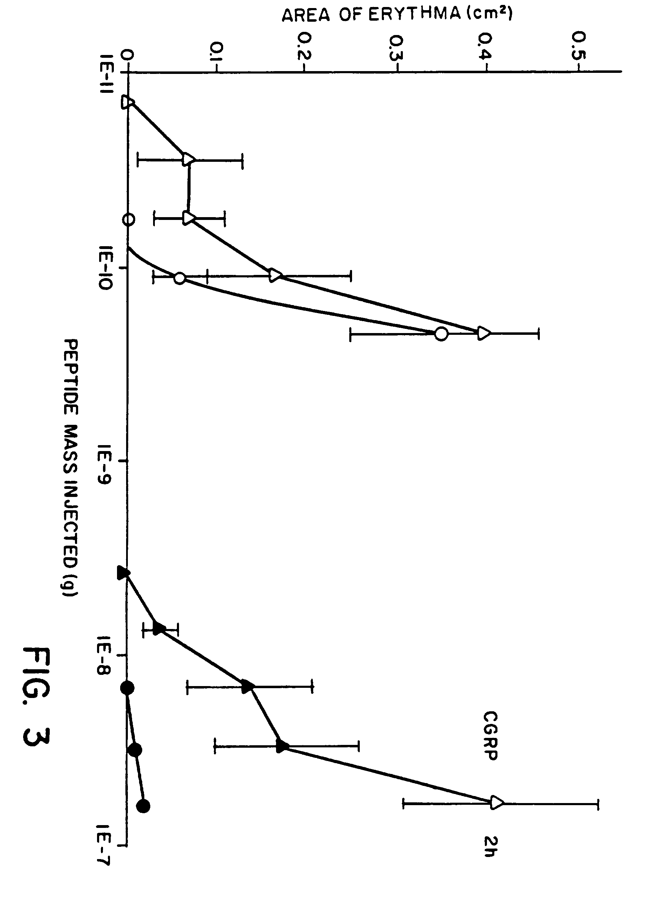

The vasodilatory activity of RP-HPLC purified LP as compared to synthetic human α-CGRP on rabbit skin is shown in Figure 3. The amount of LP injected into the skin was based on the area of the absorbing peak collected from RP-HPLC. The indicated amounts of RP-purified LP or synthetic CGRP, in a volume of 50 µl of PBS, were injected into the shaved back of a rabbit. The area of resultant erythema was measured at 2 hours (triangles) and again at 4 hours (circles). Bars represent average and SE of triplicate injections. Two hours after injection, 0.1 nanograms of LP gives an erythema equivalent to 50 nanograms of CGRP. At 4 hours, erythema from LP is still present whereas the CGRP injection site has only residual erythema.

### Demonstration of Vasodilatory Activity of LP

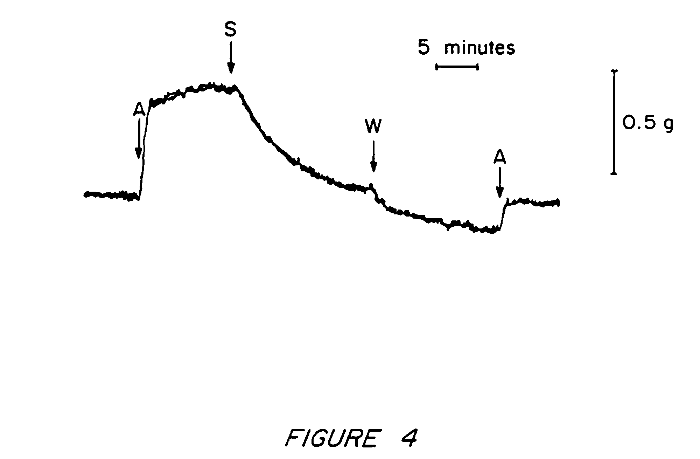

The vasodilatory activity of the LP is shown by the relaxation of a constricted rabbit aortic ring by Lutzomyia longipalpis salivary gland lysates. In FIGURE 4, A indicates the addition of adrenaline, 200 mg total; S represents the addition of the salivary gland lysate from three sand flies (approx. 30-40 ng LP); and W represents the washing of the preparation. Thoracic aortas were obtained from adult New Zealand rabbits. 4 mm-wide rings were suspended on a 3-ml bath containing Tyrode's solution bubbled with 95% O₂ and 5% CO₂ and kept at 37°C under initial tension of 1 g (Webster et al., Meth. Enzymol. 293: 531-541 (1970)).

An auxotonic pendulum level (Paton, J. Physiol. Lond. 137: 35P-36P (1957) coupled to a Harvard isotonic transducer served to measure the contractions. In four experiments, relaxation of more than 50% was achieved when homogenate from 3 pairs of glands were added to the 2.5 ml chamber (58 ± 8%, mean ± S.E.). In all cases there was a 15-30 second delay between addition of the salivary homogenate and beginning of the relaxation. After the preparation was washed, further addition of adrenaline did not restore the pre-treatment level of contraction, indicating that the activity persisted (FIGURE 4). The duration of the erythema induced by LP upon injection into mammalian skin suggests the vasodilation activity lasts at least 24 hours.

### Amino Acid Sequence Determination

HPLC-purified biologically active LP was subjected to amino acid micro-sequencing. Amino acid residues 3-13 were determined but not residues 1 or 2 of the mature protein sequence. A degenerate oligonucleotide was used in conjunction with an oligo-dT primer in the polymerase chain reaction to directly amplify a miniscule amount of LP cDNA which had been made from about 60 cells, or 1/5th of a pair of salivary glands. This amplified DNA sequence was missing the most 5' end coding for amino acids 1 and 2 and upstream sequences including the signal sequence and promoter. However, this DNA sequence yielded the nucleotide sequence 3' to the nucleotides coding for amino acid residue 14. This sequence information was used to select a genomic clone from a sand fly genomic library. A new oligonucleotide was then prepared from the genomic DNA which had the sequence from the signal peptide, along with an oligonucleotide from the 3' untranslated region of the LP cDNA.

These oligonucleotides were used as primers to amplify the cDNA coding for the complete sequence, including residues 1 and 2, which was then sequenced by standard technique. The nucleotide sequence of the cDNA and the deduced amino acid sequence of the mature LP is shown as sequence 1 in the sequence listing at page 26. The genomic LP DNA sequence, shown as sequence 2 in the sequence listing at page 26, varies somewhat from the LP cDNA sequence and is believed to represent a variant LP gene and includes the DNA sequence and deduced amino acid sequence of the 17 amino acid leader peptide. The signal sequence of LP is also given in sequence 2 (nucleotides 1-51).

### Solid Phase Peptide Synthesis

The LP peptide may be prepared conveniently using standard solid-phase peptide synthesis (Merrifield, FED. PRC. Fed. Proc. Fed. Amer. Soc. Exp. Biol. 24:412 (1962)). In such a synthesis, the solid phase support acts as a C-terminal protecting group for the growing oligomer chain. Thus, in general, N-terminal protected amino acid or peptide is reacted with a suitably functionalized and soluble polymer such that the C-terminal residue is attached to the insoluble support. The N-terminal protecting group is then selectively removed from the aminoacyl polymer and the next N-protected amino acid or peptide is coupled to the polymer using a suitable reagent for reaction of the carboxyl group of the amino acid or peptide to be introduced. The cycle of deprotection and coupling can be repeated as necessary, using the appropriate amino acid or peptide derivatives, to assemble on the polymer carrier the desired amino acid sequence of the peptide. Once the sequence is complete, a more rigorous reagent is applied to the peptide/polymer, to cleave the bond linking the peptide to the polymer, thus liberating the peptide which can be recovered using conventional techniques. Depending on the conditions used, the peptide may have a C-terminal acid or amide group and may, or may not, possess a N-terminal protecting group.

It will also be appreciated that any other reactive group(s) such as amino, carboxy, hydroxy, or mercapto group(s) if present, will have been suitably protected during the synthesis and may still be in a protected state after cleavage of the peptide from the polymer. Further processing of the peptide is therefore often necessary to obtain the desired compound.

Peptide synthesis including the introduction and removal of protective groups is well known in the art. See for example "The Peptides" Volume 3, Gross and Meienhoffer, Academic Press, 1981. The amino acid or peptide starting materials, or reactive derivatives thereof for use in the solid phase synthesis, are either known compounds, or may be prepared by methods analogous to those used for the preparation of the known compounds. Particular reagents which may be used for activation of the carboxyl group of the amino acid or peptide include for example imides such as dicyclohexylcarbodiimide.

The resin may be, for example, an insoluble polymeric support, e.g. a polyamide resin such as a cross-linked polydimethylacrylamide resin or any inert macroreticular resin such as polystyrene cross-linked with divinyl benzene or a methyl benzhydrylamine resin.

Two procedures have been found which are useful in the preparation of compounds of the invention. The first of these is the BOC procedure, where the protectant group used in the synthetic cycle is a tertiary butoxycarbonyl group. The BOC protectant group is selectively removed at each stage using trifluoroacetic acid and dichloromethane. After completion of the synthetic cycles the peptide is removed from the resin by treatment with hydrogen fluoride and anisole. The second procedure is known as the FMOC procedure and utilizes a fluorenylmethoxycarbonyl group which is selectively removed using 20% piperidine in dimethylformamide. The peptide is cleaved from the resin by treatment with trifluoroacetic acid and anisole.

Calcitonin and CGRP have a C-terminal amide group which is necessary for activity. If LP requires amidation, it may be provided by appropriate choice of the cleavage conditions used in the solid phase synthesis described above. Thus, the compound may be cleaved from the support and amidated in a one-step process by treatment with, for example, methanol and ammonia. Alternatively, where the cleavage conditions are chosen to yield a peptide with a C-terminal carboxylic acid, the amide, if necessary, may be obtained by conventional means, for example where the penultimate C-terminal residue is leucine, by enzymatic treatment with carboxypeptidase Y, and where the penultimate residue is glycine, with amidating enzyme (Bradbury, et. al., Nature 298:240-244 (1982)), or by chemical treatment of the peptide with, for example, ammonia. Alternatively solution phase peptide synthesis techniques may be used for preparation of the LP peptide.

### Recombinant Production of LP

Knowledge of the amino acid sequence of LP also permits production of the protein using recombinant DNA techniques which are well known. Thus, a gene encoding the amino acid sequence, or various analogs thereof, can be produced, for example, by oligonucleotide synthesis and subsequent ligation if necessary to form a complete coding region. The coding region may be ligated to 3' and 5' untranslated DNA regions constituting, as required, a poly A site, promoter, ribosome binding site, stop and start codons, etc. Fused DNAs, e.g., comprising DNA coding for a host polypeptide and LP polypeptide can be used for production of fusion proteins comprising LP polypeptides. The construction of an expression vector suitable for production of LP products in a selected cell type also is within the skill of the art. Culture of transformed cells results in intracellular accumulation or secretion of protein which may be purified, refolded, and otherwise post translationally modified as desired or as necessary.

### Temporary Immune Suppression

### Assay of H₂O₂ Production by Macrophages as a Marker of Immune Stimulation

Monocytes obtained by leukophoresis of healthy volunteers were purified on Ficoll-hypaque and Percoll (Pharmacia, Piscataway, NJ) gradients and placed into microtiter wells (5 x 10⁵/well) in RPMI-1640 (GIBCO, Grand Island, NY) with 5% pooled normal human serum and 1% gentamycin (M.A. Bioproducts, Walkinville, MD). The cells were greater than 95% macrophages as judged by esterase staining. (The Manual of Macrophage Methodology, Herscowitz et al., eds. Marcel Dekker, NY, p. 199 (1981)). After one day in culture, non-adherent cells were rinsed away and the macrophages were treated, e.g., with human CGRP, Calcitonin, or LP as described below. Following treatment, the macrophages were incubated for three days with a concentration of from 100 to 400 units interferon gamma (Amgen Biologicals, Thousand Oaks, CA), in medium with 15% pooled normal human serum, at which time the H₂O₂ concentration in the cells was determined.

H₂O₂ production by macrophages was determined by fluorometric assay using the fluorophore scopoletin (de la Harpe et al., J. Immunol. Methods 78: 323 (1985)). Wells containing macrophages were washed and incubated for 90 minutes with a buffered solution of scopoletin (Sigma, St. Louis, MO), PMA (Sigma, St. Louis, MO), and horseradish peroxidase (HRPO) (Sigma, St. Louis, MO). Triggered by PMA, an activated macrophage releases H₂O₂ which oxidizes scopoletin to a non-fluorescent product in a reaction catalyzed by HRPO. The amount of H₂O₂ released per culture is determined as nanomoles H₂O₂. To control for variations in cell numbers from culture to culture, the data were normalized to µg of DNA per culture; all data are presented as nanomoles H₂O₂ released/µg DNA/hour. DNA was determined by a fluorescence assay (Kissane et al., J. Biol. Chem. 233: 184 (1958).

### CGRP Inhibition of Macrophage Function

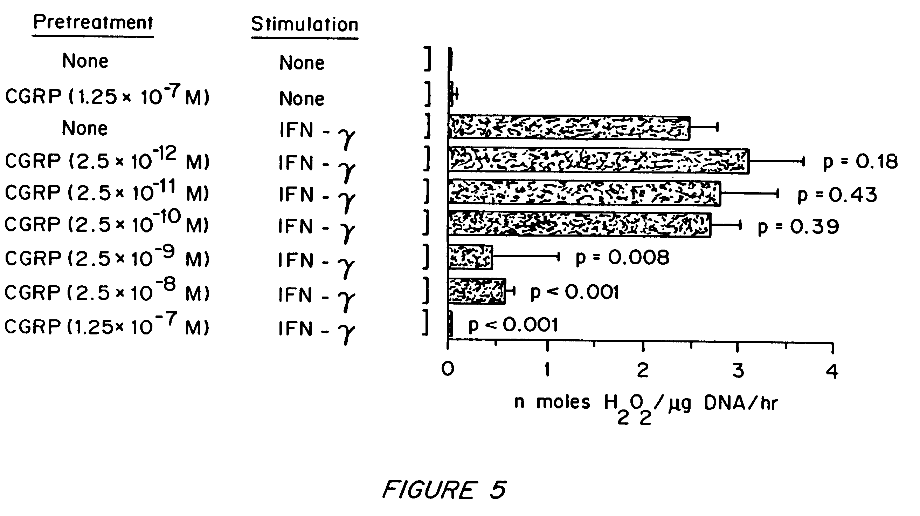

Human macrophage monolayers were pretreated for three hrs. with varying concentrations of human CGRP. The cells were activated with IFN-γ (100 to 400 units/per ml.) for 72 hrs. The amount of H₂O₂ produced by the cells was then determined according to the above procedure. The bars in FIGURE 5 represent mean H₂O₂ production for triplicate cultures ± SD. The p values were derived by comparing the mean H₂O₂ response obtained in each of the CGRP-treated groups with the H₂O₂ response obtained in the positive control cultures not treated with CGRP but stimulated with IFN-γ. Similar results were obtained in 4 replicate experiments. CGRP was found to markedly inhibit the ability of the macrophages to produce H₂O₂ response to IFN-γ (FIGURE 5). Concentrations of CGRP as low as 2.5 x 10⁻⁹ M significantly inhibited H₂O₂ production by the macrophages; higher concentrations completely abrogated the production of H₂O₂ (FIGURE 5). Results given in FIGURE 5 utilize 200 units/ml of IFN-γ, the optimal concentration to stimulate the macrophages. Similar results were obtained using the other test doses. Concentrations of IFN-γ were within 100 units/ml of elicited levels of H₂O₂ production by the macrophages which were significantly different than background values.

### Calcitonin Inhibition of Macrophage Function

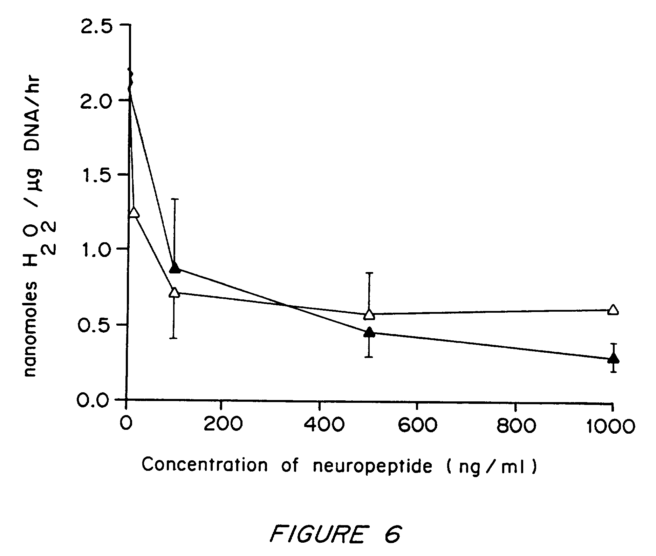

Human macrophages were treated with the amounts of either CGRP (Δ) or calcitonin (Δ) indicated in FIGURE 6, as a preincubation step for 3 hrs. The same procedure was followed as described above for CGRP. The cells were rinsed and IFN-γ (200 units/ml) was added. After 3 days incubation, H₂O₂ production was determined. The bars represent mean H₂O₂ production for triplicate cultures ±SD. Since CGRP and calcitonin are nearly identical in molecular weight, 1000 ng/ml, a concentration of 2.5 X 10⁻⁷M, was used for the two substances. Calcitonin was found to inhibit H₂O₂ production by macrophages to a degree similar to that seen with CGRP (FIGURE 6).

### LP Inhibition of Macrophage Function

Human macrophages were pretreated with varying doses of sand fly salivary gland lysates containing LP or medium for 3 hrs. the salivary material was washed away and the cells were activated with IFN-γ (200 units/ml). Three days later the amount of H₂O₂ produced by the cells in response to IFN-Y was determined as pM H₂O₂ per culture ± SD. These data were normalized to the µg of DNA/culture to control for variation in cell numbers from culture to culture. The inhibition of the IFN-γ induced H₂O₂ response of the macrophages is shown in the Table below.

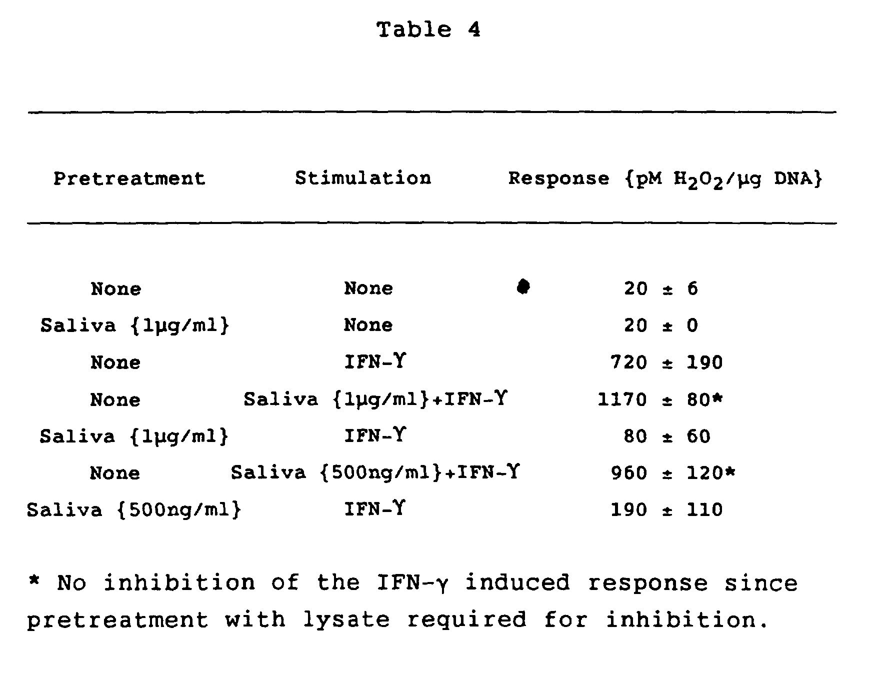

### CGRP Inhibition of Macrophage Presentation of Antigen

An OVA-specific T-cell line was produced in BALB/c mice (Titus et al., S. Immunol. 133:1594 (1984)). The line was L3T4⁺ and was maintained by successive cycles of restimulation and rest in vitro. (Kimono et al., J. Exp. Med. 152:759 (1980)). As a source antigen-presenting cells, BALB/c peritoneal cells (Titus et al., Clin. Exp. Immunol. 55:157, 1984) were placed into microtiter wells (10⁴/well) in Dulbecco's modified Eagle's medium (DMEM) (Maryanski et al., Eur. J. Immunol. 12:401 (1982) supplemented with 5% fetal calf serum (Hyclone, Logan, UT) and cultured overnight. Non-adherent cells were rinsed out of the wells, and medium with or without rat CGRP (1.25 x 10⁻⁷ M) was added as a pre-incubation step. Three hours later the wells were rinsed to remove the CGRP and the indicated number of OVA-specific T-cells (Sigma, St. Louis, MO) were added. At varying times thereafter, the wells were pulsed with lµCi³H methylthymidine (³H TdR) (Amersham, Arlington Heights, IL) and thymidine incorporation was assessed (Titus et al., J. Immunol. 133:1594 (1984)).

BALB/c peritoneal macrophages (10⁴/well) were used as antigen-presenting cells. The macrophages were preincubated in 1.25 x 10⁻⁷M rat CGRP for three hrs., and the CGRP was then washed away. OVA-specific T-cells and OVA were then added to the cultures to assist the ability of the CGRP-treated macrophages to present antigen as measured by the degree of proliferation of the T-cells. Forty-eight hours later the cultures were pulsed with ³H TdR to assess the degree of proliferation of the T-cells. The numbers in the Table below represent the mean thymidine incorporation of quadruplicate cultures ± SD. Background responses (macrophages + T-cells but no OVA, or T-cells + OVA but no macrophages) ranged between 300 to 500 CPM. Similar results were obtained with varying numbers (10³ to 2 X 10⁴/well) of peritoneal cell macrophages. The results in the Table indicate that the ability of murine macrophages to present OVA to an OVA-specific T-cell line was inhibited by CGRP. Similar results were obtained with different numbers of T-cells and different doses of OVA to stimulate the cultures. In addition, the inhibition of macrophage antigen presentation by CGRP was not due to simply delaying the kinetics of the response of the OVA-specific T-cells, since similar inhibition of proliferation of the T-cells was observed at day 2 of culture.

| Macrophages preincubated in | Number of T cells/well | Response(CPM±SD) 200mg/ml OVA | to stimulation with 400mg/ml OVA |
|---|---|---|---|
| | | | |
| Medium | 15,000 | 5,710±1,300 | 8,450±1,140 |
| CGRP | 15,000 | 1,620± 910 | 2,620± 700 |
| Medium | 30,000 | 5,230±1,150 | 10,970± 800 |
| CGRP | 30,000 | 830± 500 | 3,380±1,870 |
| | | | |

### LP Inhibition of Macrophage Presentation of Antigen

The procedure followed was the same as that for CGRP. BALB/c peritoneal macrophages (2x10⁴/well) were preincubated with medium alone (positive control) or with the indicated concentrations of L. longipalpis salivary gland lysates containing LP for 3 hrs. and rinsed free of the material. 2x10⁴ Leishmania major specific T cells and 2x10⁴ L. major were then added to the cultures. Twenty-four hrs. later the cultures were pulsed with ³H to assess the degree of proliferation of the T cells. The numbers in the Table below represent the mean thymidine incorporation of triplicate cultures ± SD.

| Macrophages preincubated in | Response {Mean ³H TdR incorporation} | % inhibition |
|---|---|---|
| Medium | 2065±621 | N/A |
| 1 gland/ml | 726±460 | 65 |
| 0.2 gland/ml | 1002±901 | 51 |
| 0.05 gland/ml | 1882±395 | 9 |

Sequence Listing

Sequence ID No.: 1
Sequence Type: Nucleotide sequence with
  corresponding amino acid sequence
Sequence Length: 315
Molecule Type: cDNA

TGT GAT GCA ACA TGC CAA TTT CGC AAG G(T)CC ATA GAT GAC TGC CAG AAG 48
Cys Asp Ala Thr Cys Gln Phe Arg Lys Ala Ile Asp Asp Cys Gln Lys

CAG GCG CAT CAT AGC AAT GTT TTG CAG ACT TCT GTA CAA ACA ACT GCA 96
Gln Ala His His Ser Asn Val Leu Gln Thr Ser Val Gln Thr Thr Ala

ACA TTC ACA TCA ATG GAT ACC TCC CAA CTA CCT GGA AAT AGT GTC TTC 144
Thr Phe Thr Ser Met Asp Thr Ser Gln Leu Pro Gly Asn Ser Val Phe

AAA GAA TGT ATG AAG CAG AAG AAA AAG GAA TTT AAG GCA GGA AAG TAA 192
Lys Glu Cys Met Lys Gln Lys Lys Lys Lys Phe Lys Ala Gly Lys

AATGATTGAA GAAAATTGTA GCCGAGGAGA GAAAGAAAGA AAGTCCCATA CCATATTTTG 252
TTTGTTAATT GTAACGAATT TTCCGAAAAA ATAAAATATT ATGCACTCAA TTTAAAAAAA 312
AAA 315

Sequence ID No.:  2
Sequence Type:  Nucleotide sequence
Sequence Length:  243
Molecule Type:  genomic DNA

ATG AAA TAT TCT TTA AAT AAT CTC CAT TTT CTT GTA GAC GTT GCT GAG     48
Met Lys Tyr Ser Leu Asn Asn Leu His Phe Leu Val Asp Val Ala Glu

GGC TGT GAT GCA ACA TGT CAA TTT CGC AAG GCC ATA GAA GAC TGC AGG     96
Gly Cys Asp Ala Thr Cys Gln Phe Arg Lys Ala Ile Glu Asp Cys Arg

AAG AAG GCG CAT CAT AGC GAT GTT TTG CAG ACT TCT GTA CAA ACA ACT     144
Lys Lys Ala His His Ser Asp Val Leu Gln Thr Ser Val Gln Thr Thr

GCA ACA TTT ACA TCA ATG GAT ACC TCC CAA CTA CCT GGA AGT GGT GTT     192
Ala Thr Phe Thr Ser Met Asp Thr Ser Gln Leu Pro Gly Ser Gly Val

TTC AAA GAA TGC ATG AAG GAG AAA GCT AAG GAA TTT AAG GCA GGA AAG     240
Phe Lys Glu Cys Met Lys Gln Lys Ala Lys Lys Phe Lys Ala Gly Lys

TAG                                                                 243

## Claims (Claims for the following Contracting State(s): AT, BE, CH, DE, DK, FR, GB, IT, LU, NL, SE)

1. A substantially pure protein, or an active fragment thereof, derivable from the salivary gland lysate of the sand fly Lutzomyia longipalpis which protein induces vasodilation or temporary immunosuppression in a mammal and has a molecular weight of about 6839 daltons as determined by mass spectrometry.

2. A protein or fragment of claim 1, wherein the protein is characterized by any of
(a) elution prior to CGRP in an acetonitrile-H₂O-trifluoroacetic acid elution in a reverse-phase high-performance liquid chromatography column; or
(b) having vasodilation activity as measured by erythema induction in animal skin at least about 80-100 times that of CGRP; or
(c) constituting about 1% of the total protein in the salivary glands of said fly.

3. A composition comprising a vasodilatory protein, or an active portion thereof, derivable from the salivary gland lysate of the sand fly Lutzomyia longipalpis and a pharmaceutically compatible carrier, which protein has a molecular weight of about 6839 daltons as determined by mass spectrometry.

4. A protein derivable from the salivary gland lysate of the sand fly Lutzomia longipalpis which inhibits macrophage function, and has a molecular weight of about 6839 daltons as determined by mass spectrometry.

5. The protein of claim 4, wherein the protein is characterised by any of
(a) elution prior to CGRP in an acetonitrile-H₂O-trifluoroacetic acid elution in a reverse-phase high-performance liquid chromatography column; or
(b) constituting about 1% of the total protein in the salivary glands of said fly.

6. A synthetic protein or peptide comprising an amino acid sequence sufficiently duplicative of the sequence of the active portion of the protein of claim 1 or claim 4 such that said protein is capable of inducing vasodilation or temporary immunosuppression in a mammal.

7. The protein of claim 6 produced (a) by expression of recombinant DNA in a host cell; or (b) by chemical peptide synthesis.

8. A vasodilatory protein having an amino acid sequence corresponding to that of the nucleotide sequence shown in the sequence listing of Sequence ID No. 1, or Sequence ID No. 2, or having an amino acid sequence corresponding to that of the nucleotide sequence shown as encoding residues 18-80 as shown in Sequence ID No. 2.

9. Nucleic acid encoding the protein according to claim 1, claim 4 or claim 6.

10. DNA encoding the vasodilatory protein of claim 8, e.g. having the nucleotide sequence depicted in the sequence listing of Sequence ID No. 1, Sequence ID No. 2 or the nucleotide sequence of 52 through 240 as shown in Sequence ID No. 2.

11. An expression vector containing the DNA of claim 10; or a cell transformed with said expression vector.

12. DNA encoding a signal sequence of a protein of claim 1.

13. DNA of claim 12, having the nucleotide sequence corresponding to nucleotide 1-51 of sequence 2 in the sequence listing.

14. A protein, an active fragment thereof or a peptide according to claim 1 or claim 6, for use in therapy, e.g.
(a) in increasing blood flow in a mammalian circulatory system by parenteral administration of the protein, active fragment or peptide, to induce vasodilation; or
(b) increasing blood flow locally in a vascular bed by topical application of the protein, active fragment or peptide, to induce vasodilation; or
(c) in inducing temporary immunosuppression in a mammal.

15. Use of a protein or peptide according to claim 4 and/or claim 6 and CGRP, calcitonin or mixtures thereof for the manufacture of a parenteral medicament for desensitising a mammal to the effects of an immunogen by temporarily suppressing the immune system of the mammal during exposure to the immunogen, the immunogen being for example an immunogen derived from a source xenotypic to said mammal, e.g. in man, streptokinase or a mouse monoclonal antibody.

## Claims (Claims for the following Contracting State(s): ES)

1. A method for producing a substantially pure protein, or an active fragment thereof, derivable from the salivary gland lysate of the sand fly Lutzomyia longipalpis which protein induces vasodilation or temporary immunosuppression in a mammal and has a molecular weight of about 6839 daltons as determined by mass spectrometry, comprising the steps of:
(a) chromatographically purifying the protein; or
(b) synthesizing a peptide having an amino-acid sequence corresponding to the protein; or
(c) expressing recombinant DNA encoding the protein in a host cell.

2. A method according to claim 1, wherein the protein is characterized by any of:
(a) elution prior to CGRP in an acetonitrile-H₂O-trifluoroacetic acid elution in a reverse-phase high-performance liquid chromatography column; or
(b) having vasodilation activity as measured by erythema induction in animal skin at least about 80-100 times that of CGRP; or
(c) constituting about 1% of the total protein in the salivary glands of said fly.

3. A method for preparing a composition comprising a vasodilatory protein, or an active portion thereof, derivable from the salivary gland lysate of the sand fly Lutzomyia longipalgis, which protein has a molecular weight of about 6839 daltons as determined by mass spectrometry, comprising the step of mixing the protein with a pharmaceutically compatible carrier.

4. A method for producing a protein derivable from the salivary gland lysate of the sand fly Lutzomyia longipalpis which inhibits macrophage function, and has a molecular weight of about 6839 daltons as determined by mass spectrometry, comprising the steps of:
(a) chromatographically purifying the protein; or
(b) synthesizing a peptide having an amino-acid sequence corresponding to the protein; or
(c) expressing recombinant DNA encoding the protein in a host cell.

5. The method according to claim 4, wherein the protein is characterized by any of:
(a) elution prior to CGRP in an acetonitrile-H₂O-trifluoroacetic acid elution in a reverse-phase high-performance liquid chromatography column; or
(b) constituting about 1% of the total protein in the salivary glands of said fly.

6. A method for producing a synthetic protein or peptide comprising an amino acid sequence sufficiently duplicative of the sequence of the active portion of the protein as defined in claim 1 or claim 4 such that said protein is capable of inducing vasodilation or temporary immunosuppression in a mammal, comprising the step of synthesizing the protein.

7. The method of claim 6 wherein the step of synthesizing the protein comprises (a) expression of recombinant DNA in a host cell; or (b) chemical peptide synthesis.

8. A method for producing a vasodilatory protein having an amino acid sequence corresponding to that of the nucleotide sequence shown in the sequence listing of Sequence ID No. 1, or Sequence ID No. 2, or having an amino acid sequence corresponding to that of the nucleotide sequence shown a encoding residues 18-80 as shown in Sequence ID No. 2, comprising the steps of:
(a) chromatographically purifying the protein; or
(b) synthesizing a peptide having an amino-acid sequence corresponding to the protein; or
(c) expressing recombinant DNA encoding the protein in a host cell.

9. A method for producing a Nucleic acid encoding the protein as defined in claim 1, claim 4 or claim 6, comprising the step of providing DNA encoding the protein, for example by oligonucleotide synthesis and ligation.

10. A method for producing DNA encoding the vasodilatory protein of claim 8, e.g. having the nucleotide sequence depicted in the sequence listing of Sequence ID No. 1, Sequence ID No. 2 or the nucleotide sequence of 52 through 240 as shown in Sequence ID No. 2, comprising the step of providing DNA encoding the protein, for example by oligonucleotide synthesis and ligation.

11. A method for producing an expression vector containing the DNA as defined in claim 10; or a cell transformed with said expression vector, comprising the step of cloning the DNA into the expression vector or transforming a cell with the expression vector, respectively.

12. A method for producing DNA encoding a signal sequence of a protein of claim 1, comprising the step of providing DNA encoding the signal sequence, for example by oligonucleotide synthesis and ligation.

13. The method according to claim 12, wherein the DNA has the nucleotide sequence corresponding to nucleotide 1-51 of sequence 2 in the sequence listing.

14. A protein, an active fragment thereof or a peptide as defined in claim 1 or claim 6, for use in therapy, e.g.
(a) in increasing blood flow in a mammalian circulatory system by parenteral administration of the protein, active fragment or peptide, to induce vasodilation; or
(b) increasing blood flow locally in a vascular bed by topical application of the protein, active fragment or peptide, to induce vasodilation; or
(c) in inducing temporary immunosuppression in a mammal.

15. Use of a protein or peptide as defined in claim 4 and/or claim 6 and CGRP, calcitonin or mixtures thereof for the manufacture of a parenteral medicament for desensitising a mammal to the effects of an immunogen by temporarily suppressing the immune system of the mammal during exposure to the immunogen, the immunogen being for example an immunogen derived from a source xenotypic to said mammal, e.g. in man, streptokinase or a mouse monoclonal antibody.

## Patentansprüche (Patentansprüche für folgende(n) Vertragsstaat(en): AT, BE, CH, DE, DK, FR, GB, IT, LU, NL, SE)

1. Ein im wesentlichen reines Protein oder aktives Fragment davon, ableitbar vom Speicheldrüsenlysat der Sandfliege Lutzomyia longipalpis, wobei das Protein bei einem Säuger Vasodilatation oder temporäre Immunsuppression verursacht und ein Molekulargewicht von etwa 6839 Dalton hat, massenspektrometrisch bestimmt.

2. Ein Protein oder Fragment gemäß Anspruch 1, wobei das Protein durch eines oder mehrere der folgenden Merkmale gekennzeichnet ist:
(a) Elution vor dem Calcitonin-Gen-verwandten Peptid (CGRP) in einer Acetonitril-H₂O-Trifluoressigsäure-Elution in einer Hochleistungsflüssigchromatographiesäule mit Umkehrphasen; oder
(b) vasodilatatorische Aktivität, gemessen durch Erytheminduktion in Tierhaut, die mindestens etwa 80 - 100 mal stärker als die des CGRP ist; oder
(c) das Protein ca. 1 % der Gesamtproteinmenge in der Speicheldrüse der Fliege ausmacht.

3. Eine Zusammensetzung, bestehend aus oder enthaltend ein vasodilatatorisches Protein oder ein aktives Fragment davon, ableitbar vom Speicheldrüsenlysat der Sandfliege Lutzomyia longipalpis und einem pharmazeutisch verträglichen Träger, wobei das Protein ein Molekulargewicht von etwa 6839 Dalton hat, massenspektrometrisch bestimmt.

4. Ein vom Speicheldrüsenlysat der Sandfliege Lutzomyia longipalpis ableitbares Protein, das die Makrophagenfunktion inhibiert und ein Molekulargewicht von etwa 6839 Dalton hat, massenspektrometrisch bestimmt.

5. Das Protein gemäß Anspruch 4, wobei das Protein durch eines oder mehrere der folgenden Merkmale gekennzeichnet ist:
(a) Elution vor CGRP in einer Acetonitril-H₂O-Trifluoressigsäure-Elution in einer Hochleistungsflüssigchromatographiesäule mit Umkehrphasen; oder
(b) das Protein ca. 1 % der Gesamtproteinmenge in der Speicheldrüse der Fliege ausmacht.

6. Ein synthetisches Protein oder Peptid, bestehend aus oder enthaltend eine Aminosäuresequenz mit ausreichender Duplikationsfähigkeit der Sequenz des aktiven Teils des Proteins gemäß Anspruch 1 oder Anspruch 4, so daß das Protein in der Lage ist, bei einem Säuger Vasodilatation oder temporäre Immunsuppression herbeizuführen.

7. Das Protein gemäß Anspruch 6, hergestellt durch (a) Expression der rekombinanten DNA in einer Wirtszelle; oder (b) chemische Peptidsynthese.

8. Ein vasodilatatorisches Protein mit einer Aminosäuresequenz, die der Nucleotidsequenz entspricht wie in der Sequenzliste der Sequenz Nr. 1 oder Sequenz Nr. 2 dargestellt, oder mit einer Aminosäuresequenz, die der der Nucleotidsequenz entspricht, die als codierende Reste 18-80 in Sequenz Nr. 2 dargestellt ist.

9. Eine das Protein gemäß Anspruch 1, Anspruch 4 oder Anspruch 6 codierende Nucleinsäure.

10. Eine das vasodilatatorische Protein gemäß Anspruch 8 codierende DNA, die z. B. die in der Sequenzliste der Sequenz Nr. 1 oder Sequenz Nr. 2 aufgeführte Nucleotidsequenz oder die in Sequenz Nr. 2 dargestellte Nucleotidsequenz 52 bis 240 hat.

11. Ein Expressionsvektor, der die DNA gemäß Anspruch 10 enthält; oder eine mit diesem Expressionsvektor transformierte Zelle.

12. Eine für eine Signalsequenz eines Proteins gemäß Anspruch 1 codierende DNA.

13. Die DNA gemäß Anspruch 12, wobei die Nucleotidsequenz dem Nucleotid 1 bis 51 der Sequenz 2 in der Sequenzliste entspricht.

14. Ein Protein, ein aktives Fragment davon oder ein Peptid gemäß Anspruch 1 oder Anspruch 6 zur therapeutischen Verwendung, z. B. zur
(a) Steigerung der Durchblutung im Kreislaufsystem eines Säugers durch parenterale Applikation des Proteins, aktiven Fragments oder Peptids, um Vasodilatation herbeizuführen; oder
(b) lokalen Steigerung der Durchblutung in einem Gefäßbett durch äußerliche Applikation des Proteins, aktiven Fragments oder Peptids, um eine Vasodilatation herbeizuführen; oder
(c) Induktion einer temporären Immunsuppression bei einem Säuger.

15. Verwendung eines Proteins oder Peptids gemäß Anspruch 4 und/oder Anspruch 6 und CGRP, Calcitonins oder Gemischen davon zur Herstellung eines parenteralen Medikaments zur Desensibilisierung eines Säugers gegenüber der Wirkung eines Immunogens durch temporäre Unterdrückung des Immunsystems des Säugers während der Immunogenexposition, wobei das Immunogen z. B. aus einer für den betreffenden Säuger xenotypischen Quelle abgeleitet ist, z. B. beim Menschen Streptokinase oder einem monoklonalen Mausantikörper.

## Patentansprüche (Patentansprüche für folgende(n) Vertragsstaat(en): ES)

1. Verfahren zur Herstellung eines im wesentlichen reinen Proteins oder eines aktiven Fragments davon, ableitbar vom Speicheldrüsenlysat der Sandfliege Lutzomyia longipalpis, wobei das Protein bei einem Säuger Vasodilatation oder temporäre Immunsuppression verursacht und ein Molekulargewicht von etwa 6839 Dalton hat, massenspektrometrisch bestimmt, umfassend die Schritte:
(a) chromatographische Reinigung des Proteins; oder
(b) Synthese eines Peptids, dessen Aminosäuresequenz dem Protein entspricht; oder
(c) Expression der das Protein codierenden, rekombinanten DNA in einer Wirtszelle.

2. Verfahren gemäß Anspruch 1, wobei das Protein durch eines oder mehrere der folgenden Merkmale gekennzeichnet ist:
(a) Elution vor dem Calcitonin-Gen-verwandten Peptid (CGRP) in einer Acetonitril-H₂O-Trifluoressigsäure-Elution in einer Hochleistungsflüssigchromatographiesäule mit Umkehrphasen; oder
(b) vasodilatatorische Aktivität, gemessen durch Erytheminduktion in Tierhaut, die mindestens etwa 80 - 100 mal stärker als die des CGRP ist; oder
(c) das Protein ca. 1 % der Gesamtproteinmenge in der Speicheldrüse der Fliege ausmacht.

3. Verfahren zur Herstellung einer Zusammensetzung, bestehend aus oder enthaltend ein vasodilatatorisches Protein oder ein aktives Fragment davon, ableitbar vom Speicheldrüsenlysat der Sandfliege Lutzomyia longipalpis, wobei das Protein ein Molekulargewicht von etwa 6839 Dalton hat, massenspektrometrisch bestimmt, bestehend aus oder enthaltend den Schritt des Mischens des Proteins mit einem pharmazeutisch verträglichen Träger.

4. Verfahren zur Herstellung eines vom Speicheldrüsenlysat der Sandfliege Lutzomyia longipalpis ableitbaren Proteins, das die Makrophagenfunktion inhibiert und ein Molekulargewicht von etwa 6839 Dalton hat, massenspektrometrisch bestimmt, umfassend die Schritte:
(a) chromatographische Reinigung des Proteins; oder
(b) Synthese eines Peptids, dessen Aminosäuresequenz dem Protein entspricht; oder
(c) Expression der das Protein codierenden, rekombinanten DNA in einer Wirtszelle.

5. Verfahren gemäß Anspruch 4, wobei das Protein durch eines oder mehrere der folgenden Merkmale gekennzeichnet ist:
(a)Elution vor dem CGRP in einer Acetonitril-H₂O-Trifluoressigsäure-Elution in einer Hochleistungsflüssigchromatographiesäule mit Umkehrphasen; oder
(b) das Protein ca. 1 % der Gesamtproteinmenge in der Speicheldrüse der Fliege ausmacht.

6. Verfahren zur Herstellung eines synthetischen Proteins oder Peptids, bestehend aus oder enthaltend eine Aminosäuresequenz mit ausreichender Duplikationsfähigkeit der Sequenz des aktiven Teils des Proteins gemäß Anspruch 1 oder Anspruch 4, so daß das Protein in der Lage ist, bei einem Säuger Vasodilatation oder temporäre Immunsuppression herbeizuführen, bestehend aus oder enthaltend den Schritt der Synthetisierung des Proteins.

7. Verfahren gemäß Anspruch 6, wobei der Schritt der Synthese des Proteins umfaßt (a) die Expression der rekombinanten DNA in einer Wirtszelle; oder (b) die chemische Peptidsynthese.

8. Verfahren zur Herstellung eines vasodilatatorischen Proteins mit einer Aminosäuresequenz, die der Nucleotidsequenz entspricht wie in der Sequenzliste der Sequenz Nr. 1 oder Sequenz Nr. 2 dargestellt, oder mit einer Aminosäuresequenz, die der der Nucleotidsequenz entspricht, die als codierende Reste 18-80 in Sequenz Nr. 2 dargestellt ist, umfassend die Schritte:
(a) chromatographische Reinigung des Proteins; oder
(b) Synthese eines Peptids, dessen Aminosäuresequenz dem Protein entspricht; oder
(c) Expression der das Protein codierenden, rekombinanten DNA in einer Wirtszelle.

9. Verfahren zur Herstellung einer das Protein gemäß Anspruch 1, Anspruch 4 oder Anspruch 6 codierenden Nucleinsäure, umfassend den Schritt der Bereitstellung der das Protein codierenden DNA, z. B. durch Oligonucleotidsynthese und Ligation.

10. Verfahren zur Herstellung einer das vasodilatatorische Protein gemäß Anspruch 8 codierenden DNA, die z. B. die in der Sequenzliste der Sequenz Nr. 1 oder Sequenz Nr. 2 aufgeführte Nucleotidsequenz oder die in Sequenz Nr. 2 dargestellte Nucleotidsequenz 52 bis 240 hat, umfassend den Schritt der Bereitstellung der das Protein codierenden DNA, z. B. durch Oligonucleotidsynthese und Ligation.

11. Verfahren zur Herstellung eines Expressionsvektors, der die DNA gemäß Anspruch 10 enthält; oder einer mit diesem Expressionsvektor transformierten Zelle, umfassend den Schritt der Klonierung der DNA in den Expressionsvektor oder der Transformation einer Zelle mit dem Expressionsvektor.

12. Verfahren zur Herstellung einer eine Signalsequenz eines Proteins gemäß Anspruch 1 codierenden DNA, umfassend den Schritt der Bereitstellung der die Signalsequenz codierenden DNA, z. B. durch Oligonucleotidsynthese und Ligation.

13. Verfahren gemäß Anspruch 12, wobei die Nucleotidsequenz der DNA dem Nucleotid 1 bis 51 der Sequenz 2 in der Sequenzliste entspricht.

14. Ein Protein, ein aktives Fragment davon oder ein Peptid gemäß Anspruch 1 oder Anspruch 6 zur therapeutischen Verwendung, z. B. zur
(a) Steigerung der Durchblutung im Kreislaufsystem eines Säugers durch parenterale Applikation des Proteins, aktiven Fragments oder Peptids, um eine Vasodilatation herbeizuführen; oder
(b) lokalen Steigerung der Durchblutung in einem Gefäßbett durch äußerliche Applikation des Proteins, aktiven Fragments oder Peptids, um eine Vasodilatation herbeizuführen; oder
(c) Induktion einer temporären Immunsuppression bei einem Säuger.

15. Verwendung eines Proteins oder Peptids gemäß Anspruch 4 und/oder Anspruch 6 und CGRP, Calcitonins oder Gemischen davon zur Herstellung eines parenteralen Medikaments zur Desensibilisierung eines Säugers gegenüber der Wirkung eines Immunogens durch temporäre Unterdrückung des Immunsystems des Säugers während der Immunogenexposition, wobei das Immunogen z. B. aus einer für den betreffenden Säuger xenotypischen Quelle abgeleitet ist, z. B. beim Menschen Streptokinase oder einem monoklonalen Mausantikörper.

## Revendications (Revendications pour l'(les) Etat(s) contractant(s) suivant(s): AT, BE, CH, DE, DK, FR, GB, IT, LU, NL, SE)

1. Protéine pratiquement pure, ou fragment actif de celle-ci, qui peut être obtenue à partir d'un lysat de glande salivaire de la mouche de sable Lutzomyia longipalpis, laquelle protéine induit une vasodilatation ou une immunosuppression temporaire chez un mammifère et possède un poids moléculaire, déterminé par spectroscopie de masse, d'environ 6839 daltons.

2. Procédé selon la revendication 1, dans lequel la protéine est caractérisée par l'un des traits suivants:
(a) une élution avant la CGRP dans une solution d'élution constituée d'un mélange acétonitrile-H₂O-acide trifluoroacétique sur une colonne de chromatographie liquide à haute performance en phase inversée; ou
(b) une activité vasodilatatrice, mesurée par l'induction d'un érythème sur la peau d'un animal, au moins environ 80 - 100 fois plus forte que dans le cas de la CGRP; ou
(c) le fait qu'elle représente environ 1% des protéines totales des glandes salivaires de ladite mouche.

3. Composition comprenant une protéine vasodilatatrice, ou un fragment actif de celle-ci, qui peut être obtenue à partir d'un lysat de glande salivaire de la mouche de sable Lutzomyia longipalpis, et dont le poids moléculaire, déterminé par spectroscopie de masse, est d'environ 6839 daltons, et un support compatible sur le plan pharmaceutique.

4. Protéine qui peut être obtenue à partir d'un lysat de glande salivaire de la mouche de sable Lutzomyia longipalpis, qui inhibe la fonction des macrophages et dont le poids moléculaire, déterminé par spectroscopie de masse, est d'environ 6839 daltons.

5. Protéine selon la revendication 4, caractérisée par l'un des traits suivants:
(a) une élution avant la CGRP dans une solution d'élution constituée d'un mélange acétonitrile-H₂O-acide trifluoroacétique sur une colonne de chromatographie liquide à haute performance en phase inversée; ou
(b) le fait qu'elle constitue environ 1% des protéines totales des glandes salivaires de ladite mouche.

6. Protéine ou peptide synthétique comprenant une séquence d'acides aminés suffisamment duplicative de la séquence de la partie active de la protéine définie à la revendication 1 ou 4 pour que ladite protéine soit susceptible d'induire une vasodilatation ou une immunosuppression temporaire chez un mammifère.

7. Protéine selon la revendication 6, produite (a) par expression d'ADN recombinant dans une cellule hôte; ou (b) par la synthèse chimique d'un peptide.

8. Protéine vasodilatatrice possédant une séquence d'acides aminés correspondant à celle de la séquence nucléotidique montrée dans la Séquence ID N° 1 ou dans la Séquence ID N°2 de la liste de séquences, ou dont la séquence d'acides aminés correspond à celle de la séquence nucléotidique codant pour les résidus 18 à 80 de la séquence ID N°2.

9. Acide nucléique codant pour la protéine selon la revendication 1, 4 ou 6.

10. Molécule d'ADN codant pour la protéine vasodilatatrice de la revendication 8, par exemple possédant la séquence nucléotidique décrite dans la Séquence ID N° 1 ou dans la Séquence ID N°2 de la liste de séquences, ou la séquence des nucléotides 52 à 240 de la Séquence ID N° 2.

11. Vecteur d'expression contenant l'ADN défini à la revendication 10; ou cellule transformée avec ledit vecteur d'expression.

12. Molécule d'ADN codant pour une séquence signal d'une protéine selon la revendication 1.

13. Molécule d'ADN selon la revendication 12 dans laquelle la séquence nucléotidique correspond à la séquence de nucléotides 1-51 de la séquence 2 de la liste de séquences.

14. Protéine, fragment actif de celle-ci ou peptide défini à la revendication 1 ou 6, destiné à être utilisé dans un procédé thérapeutique destiné, par exemple
(a) à augmenter le débit sanguin dans le système circulatoire d'un mammifère par administration parentérale de la protéine, d'un fragment actif ou d'un peptide de manière à induire une vasodilatation; ou
(b) à augmenter localement le débit sanguin dans un lit vasculaire par une application topique de la protéine, du fragment actif ou du peptide pour induire une vasodilatation; ou
(c) à induire une immunosuppression temporaire chez un mammifère.

15. Utilisation d'une protéine ou d'un peptide comme défini à la revendication 4 et/ou 6 et d'une CGRP, de calcitonine ou de mélanges de ceux-ci dans la fabrication d'un médicament utilisé par administration parentérale destiné à désensibiliser un mammifère vis-à-vis des effets d'un immunogène par une suppression temporaire du système immunitaire du mammifère pendant l'exposition à l'immunogène, l'immunogène étant par exemple un immunogène dérivé d'une source xénotypique pour ledit mammifère, par exemple chez l'homme, la streptokinase ou un anticorps monoclonal de souris.

## Revendications (Revendications pour l'(les) Etat(s) contractant(s) suivant(s): ES)

1. Procédé de production d'une protéine pratiquement pure, ou d'un fragment actif de celle-ci, qui peut être obtenue à partir d'un lysat de glande salivaire de la mouche de sable Lutzomyia longipalpis, laquelle protéine induit une vasodilatation ou une immunosuppression temporaire chez un mammifère et possède un poids moléculaire, déterminé par spectroscopie de masse, d'environ 6839 daltons, comprenant les étapes consistant à:
(a) purifier la protéine par chromatographie; ou
(b) synthétiser un peptide dont la séquence d'acides aminés correspond à la protéine; ou
(c) exprimer un ADN recombinant codant pour la protéine dans une cellule hôte.

2. Procédé selon la revendication 1, dans lequel la protéine est caractérisée par l'une des caractéristiques suivantes:
(a) une élution avant la CGRP dans une solution d'élut ion constituée d'un mélange acétonitrile-H₂O-acide trifluoroacétique sur une colonne de chromatographie liquide à haute performance en phase inversée; ou
(b) l'existence d'une activité vasodilatatrice, mesurée par l'induction d'un érythème sur la peau d'un animal, au moins environ 80 - 100 fois plus forte que la CGRP; ou
(c) le fait qu'elle représente environ 1% des protéines totales des glandes salivaires de ladite mouche.

3. Procédé de préparation d'une composition comprenant une protéine vasodilatatrice, ou un fragment actif de celle-ci, qui peut être obtenue à partir d'un lysat de glande salivaire de la mouche de sable Lutzomyia longipalpis, et dont le poids moléculaire, déterminé par spectroscopie de masse, est d'environ 6839 daltons, comprenant l'étape consistant à mélanger la protéine avec un support compatible sur le plan pharmaceutique.

4. Procédé de production d'une protéine qui peut être obtenue à partir d'un lysat de glande salivaire de la mouche de sable Lutzomyia longipalpis, qui inhibe la fonction des macrophages et dont le poids moléculaire, déterminé par spectroscopie de masse, est d'environ 6839 daltons, comprenant les étapes consistant à:
(a) purifier la protéine par chromatographie; ou
(b) synthétiser un peptide dont la séquence d'acides aminés correspond à la protéine; ou
(c) exprimer un ADN recombinant codant pour la protéine dans une cellule hôte.

5. Procédé selon la revendication 4, dans lequel la protéine est caractérisée par l'une des caractéristiques suivantes:
(a) une élution avant la CGRP dans une solution d'élution constituée d'un mélange acétonitrile-H₂O-acide trifluoroacétique sur une colonne de chromatographie liquide à haute performance en phase inversée; ou
(b) le fait qu'elle constitue environ 1% des protéines totales des glandes salivaires de ladite mouche.

6. Procédé de production d'une protéine ou d'un peptide synthétique comprenant une séquence d'acides aminés suffisamment duplicative de la séquence de la partie active de la protéine définie à la revendication 1 ou 4 tel que ladite protéine soit susceptible d'induire une vasodilatation ou une immunosuppression temporaire chez un mammifère, comprenant l'étape consistant à synthétiser la protéine.

7. Procédé de la revendication 6, dans lequel l'étape de synthèse de la protéine comprend (a) l'expression d'ADN recombinant dans une cellule hôte; ou (b) la synthèse chimique d'un peptide.

8. Procédé de production d'une protéine vasodilatatrice possédant une séquence d'acides aminés correspondant à celle de la séquence nucléotidique montrée dans la Séquence ID N° 1, ou la Séquence ID N°2 de la liste de séquences, ou dont la séquence d'acides aminés correspond à celle de la séquence nucléotidique codant pour les résidus 18 à 80 de la Séquence ID N°2, comprenant les étapes consistant à:
(a) purifier la protéine par chromatographie; ou
(b) synthétiser un peptide dont la séquence d'acides aminés correspond à la protéine; ou
(c) exprimer un ADN recombinant codant pour la protéine dans une cellule hôte.

9. Procédé de production d'un acide nucléique codant pour la protéine définie à la revendication 1, 4 ou 6, comprenant l'étape consistant à fournir une molécule d'ADN codant pour la protéine, par exemple par synthèse et ligature d'un oligonucléotide.

10. Procédé de production d'une molécule d'ADN codant pour la protéine vasodilatatrice de la revendication 8, par exemple possédant la séquence nucléotidique décrite dans la Séquence ID N° 1 ou dans la Séquence ID N° 2 de la liste de séquences, ou la séquence des nucléotides 52 à 240 de la Séquence ID N°2, comprenant les étapes consistant à fournir l'ADN codant pour la protéine, par exemple par synthèse et ligature d'un oligonucléotide.

11. Procédé de production d'un vecteur d'expression contenant l'ADN défini à la revendication 10; ou d'une cellule transformée avec ledit vecteur d'expression comprenant l'étape consistant à cloner l'ADN dans le vecteur d'expression ou à transformer une cellule avec le vecteur d'expression, respectivement.

12. Procédé de production d'une molécule d'ADN codant pour une séquence signal d'une protéine selon la revendication 1, comprenant l'étape consistant à fournir une molécule d'ADN codant pour la séquence signal, par exemple par synthèse et ligature d'un oligonucléotide.

13. Procédé selon la revendication 12 dans lequel la molécule d'ADN possède une séquence nucléotidique correspondant à la séquence de nucléotides 1-51 de la séquence 2 de la liste de séquences.

14. Protéine, fragment actif de celle-ci ou peptide tel que défini à la revendication 1 ou 6, destiné à être utilisé dans un procédé thérapeutique destiné, par exemple
(a) à augmenter le débit sanguin dans le système circulatoire par administration parentérale de la protéine, d'un fragment actif ou d'un peptide de manière à induire une vasodilatation; ou
(b) à augmenter localement le débit sanguin dans un lit vasculaire par une application topique de la protéine, du fragment actif ou du peptide pour induire une vasodilatation; ou
(c) à induire une immunosuppression temporaire chez un mammifère.

15. Utilisation d'une protéine ou d'un peptide comme défini à la revendication 4 et/ou 6 et d'une CGRP, de calcitonine ou de mélanges de ceux-ci dans la fabrication d'un médicament utilisé par administration parentérale destiné à désensibiliser un mammifère vis-à-vis des effets d'un immunogène par une suppression temporaire du système immunitaire du mammifère pendant l'exposition à l'immunogène, l'immunogène étant par exemple un immunogène dérivé d'une source xénotypique pour ledit mammalien, par exemple chez l'homme, la streptokinase ou un anticorps monoclonal de souris,
